# EUROPEAN PATENT SPECIFICATION

(11) **EP 4 268 893 B1**
(45) Date of publication and mention of the grant of the patent: **22.10.2025**
(21) Application number: 21910791.9
(22) Date of filing: 21.12.2021
(51) Int. Cl.: A61P 17/16, A61K 9/08, A61K 47/04, A61K 47/12, A61K 47/22, A61K 47/26, A61K 31/7028, A23L 2/00, A23L 2/52, A23L 2/68, A23L 5/00, A23L 33/125

(54) **ETHYL GLUCOSIDE-CONTAINING COMPOSITION AND METHOD FOR REDUCING A PUNGENT TASTE ATTRIBUTED TO ETHYL GLUCOSIDE**
ETHYLGLUCOSIDHALTIGE ZUSAMMENSETZUNG UND VERFAHREN ZUR VERRINGERUNG EINES SCHARFENGESCHMACKS IM ZUSAMMENHANG MIT ETHYLGLUCOSID
COMPOSITION CONTENANT DU GLUCOSIDE D'ÉTHYLE ET PROCÉDÉ POUR FAIRE DÉCROÎTRE LE GOÛT PIQUANT ATTRIBUÉ AU GLUCOSIDE D'ÉTHYLE

(30) Priority: 22.12.2020 JP 2020212430
(43) Date of publication of application: 01.11.2023
(73) Proprietor: Suntory Holdings Limited, Osaka 530-8203 (JP)
(72) Inventor: FUJITA, Yohei, Soraku-gun, Kyoto 619-0284 (JP); YOSHII, Takaaki, Soraku-gun, Kyoto 619-0284 (JP)
(74) Representative: Vossius & Partner Patentanwälte Rechtsanwälte mbB
(86) International application number: PCT/JP2021/047359
(87) International publication number: WO 2022/138651

(56) References cited:
- JP-A- 2002 348 242
- JP-A- 2005 130 710
- JP-A- 2005 314 310
- JP-A- 2017 093 341
- JP-A- H11 292 751
- OTAKA, YOICHI; YAMANOUCHI, ATSU: "Studies on the Constituents of Saké. Part 3. Acid Fraction", NIPPON NOGEIKAGAKU KAISHI, vol. 29, no. 11, 1 January 1955 (1955-01-01), JP , pages 880 - 883, XP009537907, ISSN: 0002-1407, DOI: 10.1271/nogeikagaku1924.29.880
- A ALCAZAR, P L FERNáNDEZ-CáCERES, M J MARTíN, F PABLOS, A G GONZáLEZ: "Ion chromatographic determination of some organic acids, chloride and phosphate in coffee and tea", TALANTA, ELSEVIER, AMSTERDAM, NL, vol. 61, no. 2, 1 October 2003 (2003-10-01), NL , pages 95 - 101, XP055644442, ISSN: 0039-9140, DOI: 10.1016/S0039-9140(03)00244-3
- OKA SATORU, SHIN SATO: "Contribution of Ethyl α-Ｄ-Glucoside to Flavor Construction in Sakè", BULLETIN OF THE AGRICULTURAL CHEMICAL SOCIETY OF JAPAN, vol. 50, no. 10, 1 January 1976 (1976-01-01), pages 455 - 461, XP055904225, DOI: 10.1271/nogeikagaku1924.50.10_455
- KIZAKI, YASUZO; FUKUDA, HISASHI; TAKAHASHI, KOJIRO: "Changes in Components of Kijou-shu, a Type of Saké, during Aging", JOURNAL OF THE BREWING SOCIETY OF JAPAN, vol. 93, no. 2, 15 February 1998 (1998-02-15), JP , pages 148 - 152, XP009537899, ISSN: 0914-7314, DOI: 10.6013/jbrewsocjapan1988.93.148

## Description

### TECHNICAL FIELD

The present invention relates to an ethyl glucoside-containing composition. The present invention also relates to a method of reducing the astringency from ethyl glucoside.

### BACKGROUND ART

Ethyl glucoside is a component of refined sake, wine, sweet cooking sake, vinegar, and the like. Ethyl glucoside has been reported to function to beautify the skin when orally ingested. For example, Non-Patent Literature 1 reports a skin conditioning action of ethyl-α-glucoside. According to Non-Patent Literature 2, ethyl-β-glucoside also functions to alleviate irritation from alcohol. There has been a growing need to increase the ethyl glucoside content in food and beverages.
Patent Literature 1 describes an alcoholic beverage containing ethyl glucoside and lactic acid to improve a rice-like taste and unpleasant aftertaste. Patent Literature 2 discloses a food or drink containing ethyl glucoside and glycerol for beautiful skin, and Patent Literature 3 discloses a composition containing succinic acid, malic acid, lactic acid or their salts and ethyl glucoside.

### CITATION LIST

### - Non-Patent Literature

Non-Patent Literature 1: The Society of Cosmetic Chemist of Japan, 32(1), 10-16 (1998)
Non-Patent Literature 2: J. Soc. Cosmet. Chem. Japan. Vol. 34, No. 3, 231-240 (2000)

<< Patent Literature 1: JP 2017 093341 A
Patent Literature 2: JP 2005 130710 A
Patent Literature 3: JP 2005 314310 A >>

### SUMMARY OF INVENTION

### - Technical Problem

However, increasing the concentration of ethyl glucoside in food and beverages, particularly in beverages, has been found to increase astringency from ethyl glucoside in some cases. Non-Patent Literatures 1 and 2 are silent about studies on suppression of the astringency from ethyl glucoside.

The present invention aims to provide an ethyl glucoside-containing composition having reduced astringency from ethyl glucoside, and a method of reducing the astringency from ethyl glucoside.

### - Solution to Problem

The present inventors found that use of a specific acid and/or its salt can effectively reduce astringency from ethyl glucoside.

Specifically, the present invention relates to an ethyl glucoside-containing composition and a method of reducing the astringency from ethyl glucoside as defined in the appended claims.

### - Advantageous Effects of Invention

The present invention can provide an ethyl glucoside-containing composition having reduced astringency from ethyl glucoside, and a method of reducing the astringency from ethyl glucoside.

### DESCRIPTION OF EMBODIMENTS

The ethyl glucoside-containing composition of the present invention contains ethyl glucoside, and at least one of phosphoric acid or its salt,
wherein the composition has an ethyl glucoside content of 0.4 wt% or more, and the composition has a content of the at least one of phosphoric acid or its salt, defined as a phosphoric acid content, of 0.0070 wt% or more and 1.3 wt% or less.

Herein, the ethyl glucoside-containing composition of the present invention is also referred to as the composition of the present invention.

Ethyl glucoside is a compound having a structure in which a hydroxy group attached to the carbon atom at position 1 of glucose is replaced by an ethoxy group. The glucose in ethyl glucoside may be either D-glucose or L-glucose, preferably D-glucose. Ethyl glucoside may be either ethyl-α-glucoside or ethyl-β-glucoside, or may be a combination of ethyl-α-glucoside and ethyl-β-glucoside. In the present invention, ethyl glucoside may be ethyl-α-glucoside and/or ethyl-β-glucoside. Preferably, ethyl glucoside is ethyl-α-glucoside.

The composition of the present invention has an ethyl glucoside content of 0.4 wt% or more. In one embodiment, the composition of the present invention may have an ethyl glucoside content of 99.6 wt% or less. The ethyl glucoside content of the ethyl glucoside-containing composition can be measured by high performance liquid chromatography (HPLC) .

The ethyl glucoside-containing composition of the present invention contains at least one of phosphoric acid, or its salt. A composition having an ethyl glucoside content of 0.4 wt% or more may have strong astringency from ethyl glucoside. The composition of the present invention contains the acid and/or its salt described above, so that the astringency from ethyl glucoside is reduced even when the ethyl glucoside content is 0.4 wt% or more.
Examples of an acid not forming part of the claimed invention include ascorbic acid, lactic acid, and their salts.
Examples of salts of the above acids include alkali metal salts such as sodium salt and potassium salt; and alkaline earth metal salts such as calcium salt and magnesium salt.

The acid and/or its salt is phosphoric acid and/or its salt. Use of phosphoric acid and/or its salt reduces the astringency from ethyl glucoside while an increase in acidity is suppressed. The acid and/or its salt is more preferably phosphoric acid. Examples of the salt of phosphoric acid include sodium dihydrogen phosphate, disodium phosphate, and trisodium phosphate.

The phosphoric acid is not only orthophosphoric acid. It also includes condensed phosphoric acids such as pyrophosphoric acid, polyphosphoric acid, metaphosphoric acid, tripolyphosphoric acid, tetrametaphosphoric acid, pentametaphosphoric acid, and hexametaphosphoric acid. In the present invention, any of these compounds may be used alone as the phosphoric acid, or two or more of these compounds may be used in combination as the phosphoric acid. Non-limiting examples of the salt of phosphoric acid include sodium pyrophosphate (also known as sodium diphosphate, in both hydrate and anhydrous forms (e.g., sodium diphosphate decahydrate, tetrasodium pyrophosphate (anhydrous))), acidic sodium pyrophosphate (also known as disodium dihydrogen pyrophosphate), potassium pyrophosphate (also known as tetrapotassium pyrophosphate), sodium tripolyphosphate (also known as sodium triphosphate), sodium polyphosphate, potassium polyphosphate, sodium trimetaphosphate, sodium tetrametaphosphate, sodium pentametaphosphate, potassium hexametaphosphate (also known as sodium metaphosphate (according to Japan's Specifications and Standards for Food Additives, and the like)), acidic sodium metaphosphate (also known as sodium hydrogen metaphosphate), acidic sodium hexametaphosphate, sodium ultraphosphate, potassium metaphosphate, and mixtures of these.

In one embodiment, the phosphoric acid and/or its salt is more preferably orthophosphoric acid and/or its salt.

The acid and/or its salt content of the ethyl glucoside-containing composition can be set according to the type and the like of the acid and/or its salt.

In one embodiment, the composition of the present invention is preferably free of citric acid and its salts. In one embodiment, the composition of the present invention is preferably free of acids and/or its salts other than phosphoric acid and its salt.

Preferably, the ethyl glucoside-containing composition of the present invention contains glucose. In the present invention, glucose is preferably D-glucose.

When the composition of the present invention contains glucose, the astringency from ethyl glucoside can be further reduced. At the same time, when an ethyl glucoside-containing composition in which glucose is present in a higher proportion relative to ethyl glucoside is used as an ingredient or the like to add a predetermined amount of ethyl glucoside to an oral composition such as a beverage, heavy sweetness from glucose may be disadvantageously imparted to the oral composition. When the composition of the present invention contains glucose, the glucose content relative to the total ethyl glucoside and glucose content is preferably 80 wt% or less, more preferably 75 wt% or less, and may be 1 wt% or more, preferably 20 wt% or more. In one embodiment, the glucose content of the composition cf the present invention relative to the total ethyl glucoside and glucose content is preferably 1 to 80 wt%, more preferably 20 to 75 wt%.

The glucose content of the ethyl glucoside-containing composition can be measured by HPLC.

The ethyl glucoside-containing composition of the present invention may be provided in any suitable form, for example, in the form of a solid such as power, granules, or a gel, or in the form of a liquid (including slurry). In one embodiment, the ethyl glucoside-containing composition is preferably in the form of a liquid.

When the composition of the present invention is a composition in the form of a liquid (liquid composition), the pH is preferably 3.0 to 7.0. When the composition of the present invention is in the form of a liquid, the astringency from ethyl glucoside can be reduced when the pH is 3.0 to 7.0. At a pH of more than 7.0, the saltiness and umami may be highly expressed. In order to suppress an increase in acidity, the pH is preferably 3.0 or more, more preferably 3.5 or more. In one embodiment, when the ethyl glucoside-containing composition is in the form of a liquid, preferably, the composition contains the acid and/or its salt in an amount that renders the pH to preferably 3.0 to 6.0, more preferably 3.5 to 6.0.

In one embodiment, when the acid and/or its salt is phosphoric acid and/or its salt, the pH described above is more preferably 6.0 or less and may be 5.5 or less. When the composition of the present invention contains phosphoric acid and/or its salt and has a pH of 6.0 or less, the astringency from ethyl glucoside can be more reduced. When the acid and/or its salt is phosphoric acid and/or its salt, the pH described above is more preferably 3.0 to 6.0, still more preferably 3.5 to 6.0, yet still more preferably 3.5 to 5.5. When the composition of the present invention contains phosphoric acid and/or its salt and has a pH in the above range, the astringency from ethyl glucoside can be more reduced while acidity is suppressed. In one embodiment, when the composition of the present invention is in the form of a liquid, preferably, the composition contains phosphoric acid and/or its salt in an amount that renders the pH to a value in a above range.

Herein, the pH is a value measured as 25°C. The pH can be measured by a commercially available pH meter.

When the composition of the present invention is in the form of a liquid, its Brix can be suitably set according to the application and the like of the composition of the present invention.

The Brix can be evaluated based on the Brix value obtained using a saccharometer or a refractometer. The Brix value is a value obtained by converting the refractive index measured at 20°C into the mass/mass percent of a sucrose solution based on the conversion table provided by International Commission for Uniform Methods of Sugar Analysis (ICUMSA). The Brix value is expressed by a unit "Bx", "%", or "degree". The Brix can be measured by, for example, a digital refractometer "Rx-5000" (Atago Co., Ltd.).

The ethyl glucoside-containing composition of the present invention can be used as an oral composition. Examples of the oral composition include beverages, food, pharmaceutical products for oral administration, quasi-pharmaceutical products for oral administration, and ingredients for use in production of these products. In one embodiment, the composition of the present invention can be used an ingredient containing ethyl glucoside to add ethyl glucoside to oral compositions such as beverages and food.

In one embodiment, the composition of the present invention can be provided in the form of a beverage. The beverage is usually in the form of a liquid. The beverage is more easily spread in the oral cavity upon ingestion than an oral composition in the form of a solid, so that the astringency from ethyl glucoside tends to be more easily perceived. The composition of the present invention in which the astringency from ethyl glucoside is reduced can be suitably used as a beverage.

When the composition of the present invention is a beverage, the beverage may be a not-from-concentrate beverage or a concentrated beverage, preferably a not-from-concentrate beverage. The term "not-from-concentrate beverage" refers to a beverage that can be consumed without dilution. The term "concentrated beverage" refers to a beverage that is diluted in a solvent for drinking such as water before consumption.

When the composition of the present invention is provided as a beverage, the beverage may not be easily drinkable if the Brix is high. When provided as a beverage, for example, the ethyl glucoside-containing composition preferably has a Brix of less than 40. When provided as a beverage, the Brix of the ethyl glucoside-containing composition is preferably 0.4 to 40, more preferably 0.5 to 20, still more preferably 1 to 15, yet still more preferably 2 to 10, particularly preferably 2 to 9.

A suitable range of Brix when the ethyl glucoside-containing composition is provided as a beverage may be 9 to 14.

When the composition of the present invention is provided as a beverage (preferably not-from-concentrate beverage), the ethyl glucoside content is preferably 5.0 wt% or less. When the ethyl glucoside content is 5.0 wt% or less, the astringency from ethyl glucoside can be effectively reduced in the composition of the present invention. When ethyl glucoside-containing composition is provided as a beverage, the ethyl glucoside content is preferably 0.4 to 5.0 wt%, more preferably 0.5 to 4.0 wt%, still more preferably 0.6 to 3.0 wt%.

In one embodiment, when the composition of the present invention is a beverage having a Brix of less than 40, the phosphoric acid content is 0.0070 wt% or more, preferably 0.0073 wt% or more, and may be preferably 0.30 wt% or less. When the phosphoric acid content is more than 0.0070 wt% or more, the astringency of ethyl glucoside can be effectively reduced. A phosphoric acid content of 0.30 wt% or less is preferred because the astringency from phosphoric acid and/or its salt is rendered mild.

When the composition of the present invention contains phosphoric acid and/or its salt, the content is defined as the phosphoric acid content. The phosphoric acid content can be measured by HPLC.

When the composition of the present invention is a beverage having a Brix of 2 or more and less than 40, the phosphoric acid content is 0.0070 wt% or more,
preferably 0.0073 wt% or more, and is also preferably 0.30 wt% or less.

When the composition cf the present invention is a beverage having a Brix of 1.9 or more and less than 40, the phosphoric acid content is 0.0070 wt% or more,
preferably 0.0073 wt% or more, and is also preferably 0.30 wt% or less.

When the composition of the present invention is a beverage having a Brix of 0.4 or more and less than 40, the phosphoric acid content is 0.0070 wt% or more,
preferably 0.0073 wt% cr more, and is also preferably 0.30 wt% or less.

When the composition of the present invention is a beverage having a Brix of 0.4 or more and 2 or less, the phosphoric acid content is preferably 0.060 wt% or more, and is also preferably 0.30 wt% or less.

In another embodiment, the composition of the present invention may be in the form of a solid or in the form of a liquid having a Brix of 40 cr more, preferably in the form of a liquid having a Brix of 40 or more. Such a composition can be suitably used, for example, as an ingredient of a beverage or the like in production thereof. In one embodiment, the present invention can provide an ethyl glucoside-containing composition that can be used as an ingredient to add ethyl glucoside to an oral composition such as a beverage and that is less likely to impart the astringency from ethyl gluccside to such an oral composition.

When using such an ethyl glucoside-containing composition as an ingredient in production of beverages and the like, the composition is preferably in the form of powder or granules, or in the form of a liquid having a Brix of 40 or more, more preferably in the form of a liquid having a Brix of 40 or more, in terms of workability and the like.

When the composition of the present invention is in the form of a liquid having a Brix of 40 or more, if the Brix is more than 75, the viscosity will be high, which may reduce workability at the production site where the composition is used as an ingredient of a beverage or the like. The Brix is preferably 75 or less in terms of workability. In one embodiment, when the ethyl glucoside-containing composition is in the form of a liquid having a Brix of 40 or more, the Brix is preferably 40 to 75, more preferably 40 to 65, still more preferably 50 to 65, yet still more preferably 55 to 65.

When the composition of the present invention is in the form of a solid or in the form of a liquid having a Brix of 40 or more, the ethyl glucoside content of the composition is preferably 10 wt% or more, more preferably 20 to 90 wt%. When the composition of the present invention is a solid, the ethyl glucoside content of the composition may be 99.6 wt% or less.

In one embodiment, when the composition of the present invention is in the form of a solid or in the form of a liquid having a Brix of 40 or more and contains phosphoric acid and/or its salt, the phosphoric acid content is preferably 0.15 wt% or more and is also 1.3 wt% or less, preferably 1.27 wt% or less. When the phosphoric acid content is 0.15 wt% or more, the astringency of ethyl glucoside can be effectively reduced. A phosphoric acid content of 1.3 wt% or less is required because the acidity is rendered mild. The phosphoric acid content can be measured by HPLC.

When the composition of the present invention is in the form of a liquid having a Brix of 40 or more and 60 or less and contains phosphoric acid and/or its salt, the phosphoric acid content is 0.15 wt% or more and is also 1.3 wt% or less, preferably 1.27 wt% or less.

When the composition of the present invention is in the form of a liquid having a Brix of 40 or more and 75 or less and contains phosphoric acid and/or its salt, the phosphoric acid content is preferably 0.15 wt% or more and is also 1.3 wt% or less.

The ethyl glucoside-containing composition of the present invention may contain one or more components different from the components described above, as long as the effect of the present invention is not impaired. For example, the composition may contain one or more additives such as flavors, pigments, antioxidants, emulsifiers, preservatives, quality stabilizers, and excipients. The composition of the present invention may contain a sweetener different from glucose. The composition of the present invention may contain water. In one embodiment, when the composition of the present invention is provided as a beverage, the ethanol content may be less than 1 v/v%. When the ethanol content is less than 1 v/v%, the astringency from ethyl glucoside can be more reduced.

The ethyl glucoside-containing composition of the present invention may be produced by any method. Ethyl glucoside can be obtained, for example, by reacting glucose with ethanol. The ethyl glucoside-containing composition of the present invention can be obtained, for example, by mixing a predetermined amount of ethyl glucoside with components such as the acid and/or its salt. When the ethyl glucoside-containing composition is provided as a composition in the form of a liquid, the Brix can be adjusted by adjusting the amounts of ethyl glucoside and optional components such as glucose, diluting the composition with water, and concentrating the composition, for example.

When using the composition of the present invention as an ingredient in production of oral compositions, the composition of the present invention may be used in any oral compositions, but beverages are preferred. The astringency from ethyl glucoside is reduced in the composition of the present invention. Thus, when used as an ingredient of a beverage, the composition is less likely to impart the astringency from ethyl glucoside to the beverage.

The composition of the present invention may be an ethyl glucoside-containing composition for beverages. In one embodiment, the ethyl glucoside-containing composition of the present invention is particularly suitable as an ingredient containing ethyl glucoside for beverages and can be used to add ethyl glucoside to beverages.

The ethyl glucoside-containing composition of the present invention can be added to any beverage, such as mineral water, carbonated water, near water, or flavored water. The pH of the beverage is preferably 3.0 to 7.0, more preferably 3.0 to 6.0, still more preferably 3.0 to 5.5, yet still more preferably 3.5 to 5.5, particularly preferably 3.0 to 5.0. When the beverage has a pH in the above range, the astringency from ethyl glucoside is less likely to be imparted to the beverage when the ethyl glucoside-containing composition of the present invention is added thereto.

The amount of the ethyl glucoside-containing composition of the present invention to be added to an oral composition such as a beverage may be appropriately set according to the type of the oral composition and the amount of ethyl glucoside to be contained.

The ethyl glucoside-containing composition of the present invention can be added to an oral composition such as a beverage by any method such as premixing the composition with a raw material to be used in the oral composition or adding the composition at any step in the production process of the oral composition.

The present invention also encompasses a method of reducing the astringency from ethyl glucoside, including: adding at least one of phosphoric acid or its salt to an ethyl glucoside-containing composition having an ethyl glucoside content of 0.4 wt% or more.

Adding at least one of phosphoric acid or its salt to an ethyl glucoside-containing composition having an ethyl glucoside content of 0.4 wt% or more can reduce the astringency from ethyl glucoside in the composition. At least one of phosphoric acid or its salt can be used to reduce the astringency from ethyl glucoside in an ethyl glucoside-containing composition having an ethyl glucoside content of 0.4 wt% or more.

The ethyl glucoside-containing composition, acid and/or its salt, and preferred embodiments thereof are as described above. In one embodiment, the ethyl glucoside-containing composition is preferably in the form of a liquid. In one embodiment, preferably, the acid and/or its salt is added in an amount that renders the pH of the ethyl glucoside-containing composition to 3.0 to 7.0.

### EXAMPLES

Hereinafter, the present invention is more specifically described with reference to examples, but the present invention is not limited to these examples.

In the examples, ethyl glucoside was ethyl-α-D-glucoside (hereinafter sometimes described as EG). In the examples, glucose was D-glucose. In the examples, water was commercially available mineral water (Suntory Natural Mineral Water). Phosphoric acid was orthophosphoric acid.

The pH (25°C) was measured by a desktop pH meter (product name: pH meter F-51, Horiba, Ltd.).

The Brix was measured by, for example, a digital refractometer "Rx-5000" (Atago Co., Ltd.).

All the samples used in the sensory evaluation described below had a Brix of less than 40.

In the experiments in the examples described herein, the Brix of each sample is substantially proportional to the EG content (concentration). For example, when the EG content (concentration) is 1.0 wt%, the Brix is about 1.0, and when the EG content (concentration) is 2.0 wt%, the Brix is about 2.0.

### (Test Example 1)

EG was dissolved in water by varying the EG content (concentration) at 0.2 wt%, 0.3 wt%, 0.4 wt%, 0.5 wt%, and 2.0 wt% to prepare five aqueous solutions as samples for sensory evaluation (samples 1-1 to 1-5). These EG aqueous solutions each had a pH of 7.0.

Two panelists (panelists A and B) experienced in sensory evaluation performed sensory evaluation on the astringency of the samples prepared. In the sensory evaluation, each panelist tasted the sample (10 mL) in the mouth for five seconds, spit out the sample, and evaluated the intensity of the astringency (astringent aftertaste). When evaluating a different sample, each panelist rinsed the mouth with water until the taste in the mouth disappeared.

The astringency intensity was evaluated at nine levels with 0.5 increments according to the following evaluation criteria (1 to 5 points). Then, the scores given by the panelists were averaged.

### Astringency intensity

1 point: Not perceived at all
2 points: Slightly perceived
3 points: Perceived
4 points: Strongly perceived
5 points: Very strongly perceived

For the sensory evaluation, the panelists exchanged views in advance, and the astringency intensity of the water (free of EG) used in preparation of the samples was given 1 point as the standard. The astringency intensity of a 2.0 wt% EG aqueous solution (sample 1-5) (pH 7.0) was given 5 points.

Table 1 shows the results (scores) of the sensory evaluation given by the panelists and average scores. In the table, the column A shows scores given by the panelist A, and the column B shows scores given by the panelist B.

The astringency was clearly perceived from the samples having an EG content of 0.4 wt% or more.

**[Table 1]**

| Sample | EG (wt%) | Astringency | | |
|---|---|---|---|---|
| | | A | B | Average |
| 1-1 | 0.2 | 1 | 1 | 1.00 |
| 1-2 | 0.3 | 1.5 | 2 | 1.75 |
| 1-3 | 0.4 | 3 | 3.5 | 3.25 |
| 1-4 | 0.5 | 3.5 | 3.5 | 3.50 |
| 1-5 | 2.0 | 5 | 5 | 5.00 |

### (Example 1)

EG was dissolved in water to prepare an aqueous solution having an EG content (concentration) of 2.0 wt%. Phosphoric acid was added to the 2.0 wt% EG aqueous solution (pH 7.0) to adjust the pH to 3.5, 4.0, 4.5, 5.0, 5.5, or 6.0, whereby samples each containing EG and phosphoric acid were obtained. The samples having a pH adjusted to 3.5, 4.0, 4.5, 5.0, 5.5, and 6.0 had a phosphoric acid concentration of 0.011 wt%, 0.0090 wt%, 0.0079 wt%, 0.0075 wt%, 0.0073 wt%, and 0.0056 wt%, respectively. The sample having the pH adjusted to 5.5 had a Brix of 1.9.

### (Examples 2 and 3)

Samples each containing EG and lactic acid and having a pH of 3.5, 4.0, 4.5, 5.0, 5.5, or 6.0 were prepared as in Example 1, except that phosphoric acid was replaced by lactic acid (Example 2) or ascorbic acid (Example 3).

### (Comparative Example 1)

Samples each containing EG and citric acid and having a pH of 3.5, 4.0, 4.5, 5.0, 5.5, or 6.0 were prepared as in Example 1, except that phosphoric acid was replaced by citric acid.

### (Sensory evaluation)

Two panelists (panelists A and B) experienced in sensory evaluation performed sensory evaluation on the astringency of the samples prepared in Examples 1 to 3 and Comparative Example 1 according to the same method and criteria as in Test Example 1.

Table 2 shows the results (scores) of the sensory evaluation of the samples by the panelists. Table 3 shows average scores. Some of the samples to which citric acid was added in Comparative Example 1 had higher astringency than the 2.0 wt% EG aqueous solution (acid-free EG aqueous solution (pH 7.0), astringency intensity: 5 points) taken as the reference sample. The samples having higher astringency intensity than the 2.0 wt% EG aqueous solution (astringency intensity: 5 points) were scored 5+ points.

**[Table 2]**

| | | pH | | | | | | | | | | | |
|---|---|---|---|---|---|---|---|---|---|---|---|---|---|
| | | 3.5 | | 4.0 | | 4.5 | | 5.0 | | 5.5 | | 6.0 | |
| | Acid | A | B | A | B | A | B | A | B | A | B | A | B |
| Example 1 | Phosphoric acid | 1 | 1 | 1 | 1 | 1 | 1.5 | 2.5 | 2.5 | 2.5 | 3 | 4.5 | 4.5 |
| * Example 2 | Lactic acid | 3 | 3 | 3 | 4 | 3.5 | 4 | 4 | 4 | 4 | 4 | 4.5 | 5 |
| * Example 3 | Ascorbic acid | 3.5 | 4 | 3.5 | 4 | 3.5 | 4 | 3.5 | 3 | 2 | 3 | 2 | 3 |
| Comparative Example 1 | Citric acid | 5+ | 5+ | 5+ | 5+ | 5+ | 5+ | 5+ | 5+ | 5 | 5 | 5 | 5 |

| | | | | | | | | | | | | | |
|---|---|---|---|---|---|---|---|---|---|---|---|---|---|
| *: Reference Example | | | | | | | | | | | | | |

**[Table 3]**

| | | pH | | | | | |
|---|---|---|---|---|---|---|---|
| | Acid | 3.5 | 4.0 | 4.5 | 5.0 | 5.5 | 6.0 |
| Example 1 | Phosphoric acid | 1.00 | 1.00 | 1.25 | 2.50 | 2.75 | 4.50 |
| * Example 2 | Lactic acid | 3.00 | 3.50 | 3.75 | 4.00 | 4.00 | 4.75 |
| * Example 3 | Ascorbic acid | 3.75 | 3.75 | 3.75 | 3.25 | 2.50 | 2.50 |
| Comparative Example 1 | Citric acid | 5.00+ | 5.00+ | 5.00+ | 5.00+ | 5.00 | 5.00 |

| | | | | | | | |
|---|---|---|---|---|---|---|---|
| *: Reference Example | | | | | | | |

The results in Examples 1 to 3 show that adding phosphoric acid, lactic acid, or ascorbic acid reduced the astringency of EG. Phosphoric acid effectively suppressed the astringency in the samples having a pH of 5.5 or less (samples having a phosphoric acid concentration of 0.0073 wt% or more). In Comparative Example 1, the astringency was more strongly perceived when the samples contained a larger amount of citric acid added and had a lower pH.

### (Test Example 2)

EG, glucose, and water were mixed to prepare samples 2-1 to 2-5. The total ethyl glucoside and glucose content (concentration) of the samples 2-1 to 2-5 was 2.0 wt%, and the glucose content (wt%) relative to the total ethyl glucoside and glucose content was varied. Also, a glucose-free EG aqueous solution having an EG concentration of 2.0 wt% was prepared as a sample 2-6.

Table 4 shows the ethyl glucoside content (wt%) and the glucose content (Glc content (%)) relative to the total ethyl glucoside and glucose content of the samples 2-1 to 2-6.

Two panelists (panelists A and B) experienced in sensory evaluation performed sensory evaluation on the astringency of the samples 2-1 to 2-6 according to the same method and criteria as in Test Example 1.

Table 4 shows the results of the sensory evaluation of the samples by the panelists and average scores. The astringency was clearly perceived from the samples 2-3 to 2-5 each having a glucose content (Glc content) of 80 wt% or less relative to the total ethyl glucoside and glucose content.

**[Table 4]**

| Sample | | 2-1 | 2-2 | 2-3 | 2-4 | 2-5 | 2-6 |
|---|---|---|---|---|---|---|---|
| EG content (wt%) | | 0.2 | 0.35 | 0.4 | 0.5 | 0.6 | 2.0 |
| Glc content (%) | | 90 | 85 | 80 | 75 | 70 | 0 |
| Astringency | A | 1 | 2.5 | 3 | 3 | 3.5 | 5 |
| | B | 1 | 2 | 3 | 3.5 | 3.5 | 5 |
| | Average | 1 | 2.25 | 3.00 | 3.25 | 3.50 | 5.00 |

### (Examples 4)

A 200 mM phosphate buffer was prepared by the following method.

A sodium dihydrogen phosphate·dihydrate (Kanto Chemical Co., Inc.) was mixed with water to prepare a 200 mM sodium dihydrogen phosphate aqueous solution. A disodium phosphate·dodecahydrate (Kanto Chemical Co., Inc.) was mixed with water to prepare a 200 mM disodium phosphate solution. The 200 mM sodium dihydrogen phosphate aqueous solution was mixed with the 200 mM disodium phosphate solution, and the pH was adjusted to 5.0. The resulting mixture was used as follows.

Maltose (71.4 g) was dissolved in a 40% ethanol aqueous solution (500 mL). The resulting solution was mixed with a 200 mM phosphate buffer (2.5 mL), water (22.5 mL), and an enzyme solution (transglucosidase L "Amano", Amano Enzyme Inc.) (25 mL). The solution to which the enzyme was added was kept warm in a hot water bath at 50°C for reaction, whereby ethyl-α-D-glucoside (EG) was generated. After the reaction, the resulting enzymatic reaction solution was heated at 85°C for 10 minutes, followed by centrifugation at 8000 rpm for 15 minutes. The pH of the centrifuged EG-containing reaction solution was adjusted to 2.0, 3.0, 4.0, or 5.0 with phosphoric acid, followed by concentration in an evaporator to a Brix of 60, whereby an EG-containing composition (1) (pH 2.0; phosphoric acid content: 4.55 wt%), an EG-containing composition (2) (pH 3.0; phosphoric acid content: 1.27 wt%), an EG-containing composition (3) (pH 4.0; phosphoric acid content:0.95 wt%), and an EG-containing composition (4) (pH 5.0; phosphoric acid content: 0.55 wt%) were obtained. The ratio of ethyl glucoside to glucose in each of the EG-containing compositions (1) to (4) was as follows: EG:glucose (weight ratio) = 1:2.1 (the glucose content relative to the total ethyl glucoside and glucose content: 67.7 wt%). The phosphoric acid content, EG content, and glucose content were measured by high performance liquid chromatography (HPLC). The EG content and the glucose content, and the phosphoric acid content were measured under the following measurement conditions.

### Measurement of the EG content and the glucose content (Device)

High performance liquid chromatography: LC-2030C (Shimadzu Corporation)
Corona detector: CORONA Ultra (Thermo Fisher Scientific K.K.)

### (Measurement conditions)

Column: COSMOSIL HILIC (Nacalai Tesque, Inc., inner
diameter: 4.6 mm; length: 150 mm)
Mobile phase (A): water
Mobile phase (B): acetonitrile
Flow rate: 0.5 mL/min
Bconc.: 80%
Oven temperature: 40°C

### Measurement of phosphoric acid content (Device)

A set of HPLC available from Shimadzu Corporation was used as a device for analysis by high performance liquid chromatography.

### (Measurement conditions)

Column: SHIM-PACK SCR-102H (8 mmL ID, 300 mL) (Shimadzu GLC Ltd.)
Detector: electrical conductivity detector CDD-6A
Eluent:
   Solution A: p-toluenesulfonic acid aqueous solution (p-toluenesulfonic acid 0.951 g/water 1 L)
   Solution B: Tris buffer containing p-toluenesulfonic acid
   Flow rate: 0.8 mL/min (both the solution A and the solution B)
   Analysis time: 38 min
   Column oven temperature: 40°C

The absolute calibration curve method was used for quantification.

The solution A (p-toluenesulfonic acid aqueous solution) was prepared by dissolving p-toluenesulfonic acid (0.951 g) in water (1 L). The solution B (Tris buffer containing p-toluenesulfonic acid) was prepared by dissolving p-toluenesulfonic acid (0.951 g), Bis-Tris (4.185 g), and EDTA (0.029 g) in water 1 L.

The EG-containing compositions (1) to (4) were diluted with water to obtain samples 1 to 4. The samples 1 to 4 were EG-containing compositions containing EG, glucose, and phosphoric acid (each having an EG concentration of 2.5 wt% and a glucose concentration of 5.8 wt%). Tables 5 and 6 show the pH of the samples 1 to 4.

### (Comparative Example 2)

A 200 mM citric acid buffer was prepared by the following method.

A trisodium citrate·dihydrate (FUJIFILM Wako Pure Chemical Corporation) was mixed with water to prepare a 200 mM trisodium citrate aqueous solution. Citric acid (FUJIFILM Wako Pure Chemical Corporation) was mixed with water to prepare a 200 mM citric acid aqueous solution. The 200 mM trisodium citrate aqueous solution was mixed with the 200 mM citric acid aqueous solution, and the pH was adjusted to 5.0. The resulting mixture was used as follows.

Samples 1' to 4' were prepared as in Example 4, except that the 200 mM phosphate buffer was replaced by the 200 mM citric acid buffer and that the pH was adjusted with citric acid instead of phosphoric acid. The samples 1' to 4' were EG-containing compositions containing EG, glucose, and citric acid (each having a EG concentration of 2.5 wt% and a glucose concentration of 5.8 wt%). Tables 5 and 6 show the pH of the samples 1' to 4'.

### (Sensory evaluation)

Two panelists (panelists A and B) experienced in sensory evaluation performed sensory evaluation on the astringency and acidity of samples 1 to 4 and samples 1' to 4'. In the sensory evaluation, each panelist tasted the sample (10 mL) in the mouth for five seconds, spit out the sample, and evaluated the intensity of the astringency (astringent aftertaste). When evaluating a different sample, each panelist rinsed the mouth with water until the taste in the mouth disappeared. The astringency intensity was evaluated according to the same evaluation criteria as those for Test Example 1 (astringency intensity of water: 1; astringency intensity of 2.0 wt% EG aqueous solution: 5). The acidity intensity was evaluated at nine levels with 0.5 increments according to the following evaluation criteria (1 to 5 points). Then, the scores given by the panelists were averaged.

### Acidity intensity

1 point: Not perceived at all
2 points: Slightly perceived
3 points: Perceived
4 points: Strongly perceived
5 points: Very strongly perceived

For the sensory evaluation, the panelists exchanged views in advance, and the acidity of the water (free of EG and glucose) used in preparation of the samples was given 1 point as the standard of the acidity. The acidity intensity of the sample 1' (pH 3.0) was given 5 points.

Tables 5 and 6 show the results of the sensory evaluation (scores given by the panelists and average scores). Table 5 shows the evaluation results of the astringency, and Table 6 shows the evaluation results of the acidity.

**[Table 5]**

| | Acid | Sample | pH | Astringency | | |
|---|---|---|---|---|---|---|
| | | | | A | B | Average |
| Example 4 | Phosphoric acid | 1 | 2.0 | 1.5 | 1 | 1.25 |
| | | 2 | 3.0 | 1.5 | 1 | 1.25 |
| | | 3 | 4.0 | 1.5 | 1 | 1.25 |
| | | 4 | 5.0 | 1.5 | 1 | 1.25 |
| Comparative Example 2 | Citric acid | 1' | 2.0 | 4.5 | 4 | 4.25 |
| | | 2' | 3.0 | 4.5 | 4 | 4.25 |
| | | 3' | 4.0 | 4 | 3.5 | 3.75 |
| | | 4' | 5.0 | 3.5 | 3.5 | 3.5 |

**[Table 6]**

| | Acid | Sample | pH | Acidity | | |
|---|---|---|---|---|---|---|
| | | | | A | B | Average |
| Example 4 | Phosphoric acid | 1 | 2.0 | 4.5 | 4 | 4.25 |
| | | 2 | 3.0 | 2 | 1.5 | 1.75 |
| | | 3 | 4.0 | 1.5 | 1 | 1.25 |
| | | 4 | 5.0 | 1.5 | 1 | 1.25 |
| Comparative Example 2 | Citric acid | 1' | 2.0 | 5 | 5 | 5.00 |
| | | 2' | 3.0 | 4.5 | 4.5 | 4.50 |
| | | 3' | 4.0 | 3.5 | 3 | 3.25 |
| | | 4' | 5.0 | 2 | 2.5 | 2.25 |

The presence of phosphoric acid in the ethyl glucoside-containing composition was found to effectively reduce the astringency from ethyl glucoside. In contrast, the astringency tended to be more strongly perceived when citric acid was added than when citric acid was not added. The samples containing phosphoric acid also had a lower acidity than the samples containing citric acid.

### (Examples 5 and Comparative Example 3)

EG was dissolved in water to prepare an aqueous solution having an EG content of 5.0 wt%. The 5.0 wt% EG aqueous solution (pH 7.0) was regarded as a sample of Comparative Example 3. Phosphoric acid was added to the 5.0 wt% EG aqueous solution to adjust the pH to 4.0, whereby a sample of Example 5 containing EG and phosphoric acid was obtained.

### (Sensory evaluation)

The astringency of the samples of Example 5 and Comparative Example 3 were sensory evaluated. In this evaluation, the astringency intensity of the water (free of EG) used in preparation of the samples was given 1 point and the astringency intensity of the sample (5.0 wt% EG aqueous solution) of Comparative Example 3 was given 5 points. Other than that, two panelists (panelists A and B) experienced in sensory evaluation performed sensory evaluation according to the same method and evaluation criteria as in Test Example 1. Table 7 shows the results. The astringency from EG was suppressed in the sample containing phosphoric acid in Example 5.

**[Table 7]**

| | pH | Astringency | | |
|---|---|---|---|---|
| | | A | B | Average |
| Example 5 | 4.0 | 3 | 3.5 | 3.25 |
| Comparative Example 3 | 7.0 | 5 | 5 | 5.00 |

### (Example 6)

The centrifuged reaction solution containing EG obtained in the process of Example 4 was concentrated in an evaporator to a Brix of 60 without the pH being adjusted with phosphoric acid, whereby an EG-containing composition (5) was obtained.

The EG-containing composition (5) was diluted with water to an EG concentration of 2.5 wt%, and phosphoric acid or sodium phosphate was added thereto, whereby samples 5, 6, and 7 having the pH adjusted to 6.0, 7.0, and 8.0, respectively, were obtained.

### (Comparative Example 4)

The centrifuged reaction solution containing EG obtained in the process of Comparative Example 2 was concentrated in an evaporator to a Brix of 60 without the pH being adjusted with citric acid, whereby an EG-containing composition (6) was obtained.

The EG-containing composition (6) was diluted with water to an EG concentration of 2.5 wt%, and citric acid or sodium citrate was added thereto, whereby samples 5' and 6' having the pH adjusted to 6.0 and 7.0, respectively, were obtained.

A sample having a pH adjusted to 8.0 could not be prepared.

### (Sensory evaluation)

Two panelists (panelists A and B) experienced in sensory evaluation performed sensory evaluation on the astringency of the samples 5 to 7 and the samples 5' and 6'. In the sensory evaluation, each panelist tasted the sample (10 mL) in the mouth for five seconds, spit out the sample, and evaluated the intensity of the astringency (astringent aftertaste). When evaluating a different sample, each panelist rinsed the mouth with water until the taste in the mouth disappeared. The astringency intensity was evaluated according to the same evaluation criteria as in Test Example 1 (astringency intensity of water: 1.00; astringency intensity of 2.0 wt% EG aqueous solution: 5.00). Then, the scores given by the panelists were averaged.

**[Table 8]**

| | Acid | Sample | pH | Astringency | | | Comment |
|---|---|---|---|---|---|---|---|
| | | | | A | B | Average | |
| Example 6 | Phosphoric acid | 5 | 6.0 | 1.50 | 1.00 | 1.25 | - |
| | | 6 | 7.0 | 1.50 | 1.00 | 1.25 | Slight saltiness and umami |
| | | 7 | 8.0 | 1.50 | 1.50 | 1.50 | High expression of saltiness and umami |
| Comparative Example 4 | Citric acid | 5' | 6.0 | 3.50 | 3.50 | 3.50 | - |
| | | 6' | 7.0 | 3.50 | 3.50 | 3.50 | - |
| | | pH not adjustable to 8.0 | | | | | - |

The samples 5 to 7 having a pH of 6.0 to 8.0 also showed that the astringency from ethyl glucoside was effectively reduced when the ethyl glucoside-containing compositions contained phosphoric acid. The saltiness and umami were perceived from the samples having a pH of 7.0 or 8.0, and the saltiness and umami were highly expressed in the samples having a pH of 8.0. The saltiness and umami are preferably not so strong. Thus, in this perspective, the pH of the ethyl glucoside-containing composition is preferably 7.0 or less.

### (Example 7)

The following test was performed to examine a suitable upper limit pH of the beverages having a Brix of less than 40.

A phosphate buffer, EG, and water were mixed to prepare a sample having a pH adjusted to 7.0, a phosphate buffer concentration of 2 mM, and an EG content (concentration) of 1.0 wt%. This sample was regarded as a sample 8. Disodium phosphate was added to the sample 8, whereby samples 9, 10, and 11 having a pH adjusted to 7.5, 8.0, and 8.5, respectively, were prepared.

### (Sensory evaluation)

Two panelists (panelists A and B) experienced in sensory evaluation performed sensory evaluation on the astringency of samples 8 to 11. In the sensory evaluation, each panelist tasted the sample (10 mL) in the mouth for five seconds, spit out the sample, and evaluated the intensity of the astringency (astringent aftertaste). When evaluating a different sample, each panelist rinsed the mouth with water until the taste in the mouth disappeared. The astringency intensity was evaluated according to the same evaluation criteria as in Test Example 1 (astringency intensity of water: 1.00; astringency intensity of 2.0 wt% EG aqueous solution: 5.00). Then, the scores given by the panelists were averaged.

Other tastes (saltiness and umami) of the samples 8 to 11 were also sensory evaluated while the astringency was evaluated. The intensity of the other tastes was evaluated at nine levels with 0.5 increments according to the following evaluation criteria (1 to 5 points). Then, the scores given by the panelists were averaged.

### Intensity of the other tastes

1.00 point: Not perceived at all
2.00 points: Slightly perceived
3.00 points: Perceived
4.00 points: Strongly perceived
5.00 points: Very strongly perceived

For the sensory evaluation, the panelists exchanged views in advance, and the intensity of the other tastes of the water (free of EG and glucose) used in preparation of the samples was given 1.00 point as the standard of the other tastes. The intensity of the other tastes to a degree that makes the original taste of the beverage completely unperceivable was given 5.00 points.

Table 9 shows the results of the sensory evaluation (scores given by the panelists and average scores).

**[Table 9]**

| | Sample | Phosphate buffer concentration | EG content (wt%) | pH | Astringency | | | Other tastes (saltiness and umami) | | |
|---|---|---|---|---|---|---|---|---|---|---|
| | | | | | A | B | Average | A | B | Average |
| Example 7 | 8 | 2mM | 1.0 | 7.0 | 2.00 | 2.00 | 2.00 | 1.00 | 1.00 | 1.00 |
| | 9 | 2 mM | 1.0 | 7.5 | 1.50 | 2.00 | 1.75 | 2.00 | 3.00 | 2.50 |
| | 10 | 2 mM | 1.0 | 8.0 | 1.50 | 1.00 | 1.25 | 3.50 | 4.00 | 3.75 |
| | 11 | 2mM | 1.0 | 8.5 | 1.00 | 1.00 | 1.00 | 4.50 | 5.00 | 4.75 |

The other tastes are perceived from the beverages having a Brix of less than 40 when the pH is more than 7.0. The saltiness and umami are preferably not so intense. Thus, in this perspective, the pH of the beverage is preferably 7.0 or less. The pH may be more than 7.0 in the case of beverages for which the perception of saltiness or umami does not pose a problem or for which the perception of saltiness or umami is preferred.

### (Examination of suitable phosphoric acid concentration range in beverages having a Brix of less than 40)

The results in Example 1 show that phosphoric acid effectively suppressed the astringency in the samples having a pH of 5.5 or less. The sample having a pH of 5.5 had a phosphoric acid concentration of 0.0073 wt% and a Brix of 1.9. The results show that the phosphoric acid concentration is preferably 0.0073 wt% or more in the beverages having a Brix of less than 40 because the astringency can be effectively suppressed.

### (Examples 8 to 11)

The astringency of the beverages having a Brix of less than 40 and a pH of 7.0 were evaluated by changing the phosphate buffer concentration and the EG concentration. At the same time, a preferred upper limit of the phosphoric acid concentration was examined.

A phosphate buffer, EG, and water were mixed to prepare samples 12 to 31 each having a phosphate buffer concentration of 0 mM, 1.0 mM, 2.0 mM, 3.0 mM, or 5.0 mM and an EG content (concentration) of 0.5 wt%, 1.0 wt%, 1.5 wt%, or 2.0 wt%.

The pH of each sample was adjusted to 7.0 by adding disodium phosphate.

The EG content (concentration) was 0.5 wt% in Example 8, 1.0 wt% in Example 9, 1.5 wt% in Example 10, and 2.0 wt% in Example 11.

### (Sensory evaluation)

Two panelists (panelists A and B) experienced in sensory evaluation performed sensory evaluation on the astringency of the samples 12 to 31. In the sensory evaluation, each panelist tasted the sample (10 mL) in the mouth for five seconds, spit out the sample, and evaluated the intensity of the astringency (astringent aftertaste). When evaluating a different sample, each panelist rinsed the mouth with water until the taste in the mouth disappeared.

Each example was evaluated, taking the astringency intensity of water as 1 and taking the astringency intensity of each of the samples 12, 17, 22 and 27 each having a phosphoric acid concentration of 0 wt% and a corresponding EG content as 5.00. In other words, the standard of the astringency intensity (5.00) varies among Examples 8 to 11.

Then, the scores given by the panelists were averaged.

Tables 10 to 13 show the results of the sensory evaluation (scores given by the panelists and average scores).

**[Table 10]**

| | Sample | Phosphate buffer concentration | Phosphoric acid concentration (wt%) | EG content (wt%) | Astringency | | |
|---|---|---|---|---|---|---|---|
| | | | | | A | B | Average |
| Example 8 | 12 | 0 mM | 0 | 0.5 | 5.00 | 5.00 | 5.00 |
| | 13 | 1.0 mm | 0.060 | 0.5 | 2.50 | 2.50 | 2.50 |
| | 14 | 2.0 mM | 0.12 | 0.5 | 3.00 | 2.50 | 2.75 |
| | 15 | 3.0 mM | 0.18 | 0.5 | 3.00 | 3.00 | 3.00 |
| | 16 | 5.0 mM | 0.30 | 0.5 | 4.00 | 4.50 | 4.25 |

**[Table 11]**

| | Sample | Phosphate buffer concentration | Phosphoric acid concentration (wt%) | EG content (wt%) | Astringency | | |
|---|---|---|---|---|---|---|---|
| | | | | | A | B | Average |
| Example 9 | 17 | 0 nM | 0 | 1.0 | 5.00 | 5.00 | 5.00 |
| | 18 | 1.0 mm | 0.060 | 1.0 | 2.00 | 2.00 | 2.00 |
| | 19 | 2.0 mM | 0.12 | 1.0 | 2.50 | 2.50 | 2.50 |
| | 20 | 3.0 mM | 0.18 | 1.0 | 3.00 | 2.50 | 2.75 |
| | 21 | 5.0 mM | 0.30 | 1.0 | 4.00 | 4.50 | 4.25 |

**[Table 12]**

| | Sample | Phosphate buffer concentration | Phosphoric acid concentration (wt%) | EG content (wt%) | Astringency | | |
|---|---|---|---|---|---|---|---|
| | | | | | A | B | Average |
| Example 10 | 22 | 0 mM | 0 | 1.5 | 5.00 | 5.00 | 5.00 |
| | 23 | 1.0 mm | 0.060 | 1.5 | 1.50 | 2.00 | 1.75 |
| | 24 | 2.0 mM | 0.12 | 1.5 | 2.50 | 2.50 | 2.50 |
| | 25 | 3.0 mm | 0.18 | 1.5 | 3.00 | 2.50 | 2.75 |
| | 26 | 5.0 mM | 0.30 | 1.5 | 4.50 | 4.50 | 4.50 |

**[Table 13]**

| | Sample | Phosphate buffer concentration | Phosphoric acid concentration | EG content (wt%) | Astringency | | |
|---|---|---|---|---|---|---|---|
| | | | | | A | B | Average |
| Example 11 | 27 | 0 mM | (wt%) 0 | 2.0 | 5.00 | 5.00 | 5.00 |
| | 28 | 1.0 mm | 0.060 | 2.0 | 1.50 | 1.50 | 1.50 |
| | 29 | 2.0 mM | 0.12 | 2.0 | 2.00 | 2.00 | 2.00 |
| | 30 | 3.0 mM | 0.18 | 2.0 | 2.50 | 2.50 | 2.50 |
| | 31 | 5.0 mM | 0.30 | 2.0 | 4.50 | 4.50 | 4.50 |

The results in Examples 8 to 11 confirmed that the astringency from EG was suppressed in the beverages having a Brix of less than 40 and a pH of 7.0 and differing in EG content and phosphoric acid concentration.

The results also show that an increase in the phosphoric acid concentration results in an increase in the astringency derived from phosphoric acid. The results show that the astringency was effectively suppressed in the samples having a phosphoric acid concentration of 0.30 wt% or less. The results show that the phosphoric acid concentration is preferably 0.30 wt% or less in the beverages having a Brix of less than 40 because the astringency can be effectively suppressed in such samples.

### (Examination of suitable phosphoric acid concentration range in compositions in the form of a liquid having a Brix of 40 or more)

The results in Example 1 show that phosphoric acid effectively suppressed the astringency in the samples having a pH of 5.5 or less. The sample having a pH of 5.5 had a phosphoric acid concentration of 0.0073 wt% and a Brix of 1.9.

When a sample having a Brix of 1.9 is concentrated to provide a sample having a Brix of 40, the phosphoric acid concentration increases by 21-fold, so that the phosphoric acid concentration of the sample having a Brix of 40 is 0.15 wt%. In other words, the phosphoric acid concentration is preferably 0.15 wt% or more in the samples having a Brix of 40 in order to effectively suppress the astringency. The lower limit of a preferred phosphoric acid concentration is higher in the samples having a Brix of more than 40. Thus, the phosphoric acid concentration is preferably 0.15 wt% or more in the samples having a Brix of 40 or more.

The results in Example 4 show that the acidity was suppressed in the samples containing phosphoric acid when the pH was 3.0 or more. The phosphoric acid concentration of the sample having a pH of 3.0 was 1.27 wt%.

The samples used in Example 4 had a Brix of 60. In other words, the phosphoric acid concentration is preferably 1.27 wt% or less in the samples having a Brix of 60 in order to suppress the acidity. The upper limit of a preferred phosphoric acid concentration is lower in the samples having a Brix less than 60. Thus, the acidity can be suppressed when the phosphoric acid concentration is 1.27 wt% or less in the samples having a Brix of 40 or more and 60 or less.

## Claims

1. An ethyl glucoside-containing composition comprising:
ethyl glucoside; and
at least one of phosphoric acid or its salt,
wherein the composition has an ethyl glucoside content of 0.4 wt% or more, and
the composition has a content of the at least one of phosphoric acid or its salt, defined as a phosphoric acid content, of 0.0070 wt% or more and 1.3 wt% or less.

2. The composition according to claim 1,
wherein the composition is in the form of a liquid having a pH of 3.0 to 7.0.

3. The composition according to claim 1 or 2,
wherein the composition is in the form of a liquid having a pH of 3.5 to 6.0.

4. The composition according to any one of claims 1 to 3, further comprising glucose.

5. The composition according to claim 4,
wherein the composition has a glucose content of 80 wt% or less relative to a total ethyl glucoside and glucose content.

6. The composition according to any one of claims 1 to 5,
wherein the composition is in the form of a liquid having a Brix of 40 or more.

7. The composition according to claim 6,
wherein the composition has a Brix of 40 or more and 60 or less, and has the content of the at least one of phosphoric acid or its salt, defined as a phosphoric acid content, of 0.15 wt% or more and 1.27 wt% or less.

8. The composition according to claim 6 or 7,
wherein the ethyl glucoside content of the composition is 20 to 90 wt%.

9. The composition according to any one of claims 1 to 5,
wherein the composition is a beverage having a Brix of less than 40.

10. The composition according to claim 9,
wherein the ethyl glucoside content of the composition is 0.4 to 5.0 wt%.

11. The composition according to claim 9 or 10,
wherein the content of the at least one of phosphoric acid or its salt, defined as a phosphoric acid content, is 0.0073 or more and 0.30 wt% or less.

12. A method of reducing astringency from ethyl glucoside, comprising:
adding at least one of phosphoric acid or its salt to an ethyl glucoside-containing composition having an ethyl glucoside content of 0.4 wt% or more, such that a content of the at least one of phosphoric acid or its salt, defined as a phosphoric acid content, in the composition is 0.0070 wt% or more and 1.3 wt% or less.

## Patentansprüche

1. Eine Ethylglucosid enthaltende Zusammensetzung, umfassend:
Ethylglucosid; und
mindestens eines von Phosphorsäure oder deren Salz,
wobei die Zusammensetzung einen Ethylglucosidgehalt von 0,4 Gew.-% oder mehr aufweist und
die Zusammensetzung einen Gehalt des mindestens einen von Phosphorsäure oder deren Salz, definiert als Phosphorsäuregehalt, von 0,0070 Gew.-% oder mehr und 1,3 Gew.-% oder weniger aufweist.

2. Die Zusammensetzung nach Anspruch 1,
wobei die Zusammensetzung in Form einer Flüssigkeit mit einem pH von 3,0 bis 7,0 vorliegt.

3. Die Zusammensetzung nach Anspruch 1 oder 2,
wobei die Zusammensetzung in Form einer Flüssigkeit mit einem pH von 3,5 bis 6,0 vorliegt.

4. Die Zusammensetzung nach einem der Ansprüche 1 bis 3, ferner umfassend Glucose.

5. Die Zusammensetzung nach Anspruch 4,
wobei die Zusammensetzung einen Glucosegehalt von 80 Gew.-% oder weniger, bezogen auf einen Gesamtgehalt an Ethylglucosid und Glucose, aufweist.

6. Die Zusammensetzung nach einem der Ansprüche 1 bis 5,
wobei die Zusammensetzung in Form einer Flüssigkeit mit einem Brix-Wert von 40 oder mehr vorliegt.

7. Die Zusammensetzung nach Anspruch 6,
wobei die Zusammensetzung einen Brix-Wert von 40 oder mehr und 60 oder weniger aufweist und einen Gehalt des mindestens einen von Phosphorsäure oder deren Salz, definiert als ein Phosphorsäuregehalt, von 0,15 Gew.-% oder mehr und 1,27 Gew.-% oder weniger aufweist.

8. Die Zusammensetzung nach Anspruch 6 oder 7,
wobei der Ethylglucosidgehalt der Zusammensetzung 20 bis 90 Gew.-% beträgt.

9. Die Zusammensetzung nach einem der Ansprüche 1 bis 5,
wobei die Zusammensetzung ein Getränk mit einem Brix-Wert von weniger als 40 ist.

10. Die Zusammensetzung nach Anspruch 9,
wobei der Ethylglucosidgehalt der Zusammensetzung 0,4 bis 5,0 Gew.-% beträgt.

11. Die Zusammensetzung nach Anspruch 9 oder 10,
wobei der Gehalt des mindestens einen von Phosphorsäure oder deren Salz, definiert als Phosphorsäuregehalt, 0,0073 oder mehr und 0,30 Gew.-% oder weniger beträgt.

12. Ein Verfahren zur Verringerung der Adstringenz von Ethylglucosid, umfassend:
Zugabe mindestens eines von Phosphorsäure oder deren Salz zu einer Ethylglucosid enthaltenden Zusammensetzung mit einem Ethylglucosidgehalt von 0,4 Gew.-% oder mehr, so dass ein Gehalt des mindestens einen von Phosphorsäure oder deren Salz, definiert als ein Phosphorsäuregehalt, in der Zusammensetzung 0,0070 Gew.-% oder mehr und 1,3 Gew.-% oder weniger beträgt.

## Revendications

1. Composition contenant de l'éthylglucoside comprenant :
de l'éthylglucoside ; et
au moins l'un parmi l'acide phosphorique et un sel de celui-ci,
laquelle composition a une teneur en éthylglucoside de 0,4 % en poids ou plus, et
laquelle composition a une teneur en l'au moins un parmi l'acide phosphorique et un sel de celui-ci, définie sous forme de teneur en acide phosphorique, de 0,0070 % en poids ou plus et 1,3 % en poids ou moins.

2. Composition selon la revendication 1, laquelle composition est sous la forme d'un liquide ayant un pH de 3,0 à 7,0.

3. Composition selon la revendication 1 ou 2, laquelle composition est sous la forme d'un liquide ayant un pH de 3,5 à 6,0.

4. Composition selon l'une quelconque des revendications 1 à 3, comprenant en outre du glucose.

5. Composition selon la revendication 4, laquelle composition a une teneur en glucose de 80 % en poids ou moins par rapport à la teneur totale en éthylglucoside et en glucose.

6. Composition selon l'une quelconque des revendications 1 à 5, laquelle composition est sous la forme d'un liquide ayant un Brix de 40 ou plus.

7. Composition selon la revendication 6, laquelle composition a un Brix de 40 ou plus et 60 ou moins, et a une teneur en l'au moins un parmi l'acide phosphorique et un sel de celui-ci, définie sous forme de teneur en acide phosphorique, de 0,15 % en poids ou plus et 1,27 % en poids ou moins.

8. Composition selon la revendication 6 ou 7, dans laquelle la teneur en éthylglucoside de la composition est de 20 à 90 % en poids.

9. Composition selon l'une quelconque des revendications 1 à 5, laquelle composition est une boisson ayant un Brix inférieur à 40.

10. Composition selon la revendication 9, dans laquelle la teneur en éthylglucoside de la composition est de 0,4 à 5,0 % en poids.

11. Composition selon la revendication 9 ou 10, dans laquelle la teneur en l'au moins un parmi l'acide phosphorique et un sel de celui-ci, définie sous forme de teneur en acide phosphorique, est de 0,0073 ou plus et 0,30 % en poids ou moins.

12. Méthode de réduction de l'astringence due à l'éthylglucoside, comprenant :
l'addition d'au moins l'un parmi l'acide phosphorique et un sel de celui-ci à une composition contenant de l'éthylglucoside ayant une teneur en éthylglucoside de 0,4 % en poids ou plus, de façon que la teneur en l'au moins un parmi l'acide phosphorique et un sel de celui-ci, définie sous forme de teneur en acide phosphorique, dans la composition, soit de 0,0070 % en poids ou plus et 1,3 % en poids ou moins.
